# EUROPEAN PATENT SPECIFICATION

(11) **EP 1 201 747 B1**
(45) Date of publication and mention of the grant of the patent: **27.10.2004**
(21) Application number: 01932249.4
(22) Date of filing: 24.05.2001
(51) Int. Cl.: C12N 9/00, C12P 13/04, C12P 1/02

(54) **PROCESS FOR PRODUCING GAMMA-GLUTAMYLCYSTEINE**
VERFAHREN ZUR HERSTELLUNG VON GAMMA-GLUTAMYLCYSTEIN
PROCEDE DE PRODUCTION DE GAMMA-GLUTAMYLCYSTEINE

(30) Priority: 25.05.2000 JP 2000155121
(43) Date of publication of application: 02.05.2002
(73) Proprietor: Ajinomoto Co., Inc., Tokyo 104-8315 (JP)
(72) Inventor: NISHIUCHI, Hiroaki, C/O AJINOMOTO Co., Inc, Kawasaki-shi, Kanagawa 210-8681 (JP); SANO, Kouichiro, C/O AJINOMOTO Co., Inc, Kawasaki-shi, Kanagawa 210-8681 (JP); SUGIMOTO, Reiko, C/O AJINOMOTO Co., Inc, Kawasaki-shi, Kanagawa 210-8681 (JP); UEDA, Yoichi, C/O AJINOMOTO Co., Inc, Kawasaki-shi, Kanagawa 210-8681 (JP)
(74) Representative: Strehl Schübel-Hopf & Partner
(86) International application number: PCT/JP2001/004366
(87) International publication number: WO 2001/090310

(56) References cited:
- WO-A-00/30474
- JP-A- 6 070 752
- OHTAKE Y ET AL: "Isolation and characterization of glutathione biosynthesis-deficient mutants in Saccharomyces cerevisiae" AGRICULTURAL AND BIOLOGICAL CHEMISTRY, vol. 54, no. 12, 1990, page 3145-3150 XP001120475
- PATENT ABSTRACTS OF JAPAN & JP 04 066069 A (AJINOMOTO CO), 2 March 1992 (1992-03-02)
- PATENT ABSTRACTS OF JAPAN & JP 61 074596 A (KYOWA HAKKO KOGYO CO LTD), 16 April 1986 (1986-04-16)
- PATENT ABSTRACTS OF JAPAN & JP 08 228714 A (AJINOMOTO CO INC), 10 September 1996 (1996-09-10)
- INOUE,YOSHIHARU ET AL.: 'Molecular identification of glutathione synthetase (GSH2) gene from saccharomyces cerevisiae' BIOCHIMICA ET BIOPHYSICA ACTA vol. 1395, no. 3, 11 February 1998, pages 315 - 320, XP002945457
- YASUYUKI OOMURA ET AL.: 'Glutathione kouseisan koubo no ikushu' BIOSCIENCE TO INDUSTRY vol. 50, no. 10, 01 October 1992, pages 989 - 994, XP002945458
- OMURA,FUMIHIKO ET AL.: 'Single point mutations in met 4p impair the transcriptional repression of MET genes in saccharomyces cerevisiae' FEBS LETTERS vol. 387, no. 2-3, June 1996, pages 179 - 183, XP002945459
- GRANT,CHRIS M. ET AL.: 'Glutathione synthetase is dispensable for growth under both normal and oxidative stress conditions in the yeast saccharomyces cerevisiae due to an accumulation of the dipeptide gamma-glutamylcysteine' MOLECULAR BIOLOGY OF THE CELL vol. 8, no. 9, September 1997, pages 1699 - 1707, XP002945460
- SUGIYAMA,KEI-ICHI ET AL.: 'The Yap1p-dependent induction of glutathione synthesis in heat shock response of saccharomyces cerevisiae' THE JOURNAL OF BIOLOGICAL CHEMISTRY vol. 275, no. 20, 19 May 2000, pages 15535 - 15540, XP002945461

### SEQUENCE LISTING

<110> Ajinomoto Co., Inc.
<120> METHOD FOR PRODUCING γ-GLUTAMYLCYSTEINE
<130> EPA-53816
<150> JP 2000-155121
   <151> 2000-05-25
<160> 9
<170> PatentIn Ver. 2.0
<210> 1
   <211> 21
   <212> DNA
   <213> Artificial Sequence
<220>
   <223> Description of Artificial Sequence: primer for PCR
<400> 1
   tatgaagact gtacagtctc c 21
<210> 2
   <211> 34
   <212> DNA
   <213> Artificial Sequence
<220>
   <223> Description of Artificial Sequence: primer for PCR
<400> 2
   ccggggagct cagctaaatg gtgtacttcg ctac 34
<210> 3
   <211> 29
<212> DNA
   <213> Artificial Sequence
<220>
   <223> Description of Artificial Sequence: primer for PCR
<400> 3
   attaacccgg gttgattcgg taatctccg 29
<210> 4
   <211> 30
   <212> DNA
   <213> Artificial Sequence
<220>
   <223> Description of Artificial Sequence: primer for PC
<400> 4
   attaacccgg ggttttttag ttttgctggc 30
<210> 5
   <211> 23
   <212> DNA
   <213> Artificial Sequence
<220>
   <223> Description of Artificial Sequence: primer for PCR
<400> 5
   agctaaatgg tgtacttcgc tac 23
<210> 6
   <211> 20
   <212> DNA
   <213> Artificial Sequence
<220>
   <223> Description of Artificial Sequence: primer for PCR
<400> 6
   cagattccga gtttactgga 20
<210> 7
<211> 23
   <212> DNA
   <213> Artificial Sequence
<220>
   <223> Description of Artificial Sequence: primer for PCR
<400> 7
   agaaggaatg agcctaaaac agc 23
<210> 8
   <211> 38
   <212> DNA
   <213> Artificial Sequence
<220>
   <223> Description of Artificial Sequence: primer for PCR
<400> 8
   ggcagggaag gcaagtagct ggcattaagt gagccctc 38
<210> 9
   <211> 38
   <212> DNA
   <213> Artificial Sequence
<220>
   <223> Description of Artificial Sequence: primer for PCR
<400> 9
   gagggctcac ttaatgccag ctacttgcct tccctgcc 38

## Description

The present invention relates to yeast and yeast extract having a high γ-glutamylcysteine content as well as a method for breeding such yeast. γ-Glutamylcysteine and cysteine produced therefrom are useful in the food industry.

### Description of the Related Art

Cysteine is used for the purpose of improving flavor of foodstuffs and so forth. While the proteolysis method, semi-synthetic method and so forth are known as methods for producing cysteine, mainly used methods at present are the proteolysis method and the semi-synthetic method. In order to utilize cysteine for improving flavor of foodstuffs, natural food materials having a high cysteine content are desired. However, such natural food materials have hardly been known so far.

Glutathione, which is a tripeptide consisting of cysteine bonded with glutamic acid and glycine, is also known to be used for improving flavor of foodstuffs. Glutathione is synthesized from cysteine via γ-glutamylcysteine. However, γ-glutamylcysteine is scarcely used for improving flavor of foodstuffs.

γ-Glutamylcysteine is synthesized from cysteine and glutamic acid with the aid of γ-glutamylcysteine synthetase (GSH1). And glutathione is synthesized from γ-glutamylcysteine and glycine with the aid of glutathione synthetase (GSH2).

A yeast strain, *Saccharomyces cerevisiae* YHT178, in which the promoter of γ-glutamylcysteine synthetase gene is replaced with a strong transcriptional promoter ΔP8, was reported to produce a large amount of γ-glutamylcysteine synthetase in its cell (Yasuyuki Ootake *et al., Bioscience and Industry, vol. 50,* No. 10, pp.989-994, 1992). Further, Ootake *et al.* also reported that glutathione was not detected in a glutathione synthetase deficient strain of *Saccharomyces cerevisiae* YL1 strain, in another report (Yasuyuki Ootake *et al., Agricultural and Biological Chemistry, vol. 12,* No. 54, pp.3145-3150, 1990).

Inoue *et al*. reported gene disruption of the glutathione synthetase gene on a chromosome (Yoshiharu Inoue *et al., Biochimica et Biophysica Acta,* No. 1395, pp.315-320, 1998). This disrupted gene is considered to code for a glutathione synthetase in which amino acid residues of 1-396th positions are correctly translated, but a C-terminus region from the 397th position is deleted. Inoue *et al.* reported that glutathione content of the gene-disrupted strain was measured, but glutathione was not detected.

By the way, while it is known that a flavor composition can be obtained by adding a saccharide to γ-glutamylcysteine and heating them (Japanese Patent Laid-open Publication (Kokai) No. 4-91762), it is not known that cysteine is released when γ-glutamylcysteine is heated.

As described above, there are reports concerning enhancement of expression of γ-glutamylcysteine synthetase and disruption of glutathione synthetase gene. However, the obtained *Saccharomyces cerevisiae* strains showed a low γ-glutamylcysteine content or did not show good growth in any case, and they are not considered to fully satisfy the requirements needed for the industrial production.

It was reported that the YHT178 strain in which expression of γ-glutamylcysteine synthetase was enhanced could accumulate 1.69% of γ-glutamylcysteine at most in its cell in a synthetic minimal medium (Ootake *et al., Bioscience and Industry, supra*). However, the growth rate of the yeast in this medium was not reported. Although the growth rate in YPD medium which is more nutritious than the synthetic minimal medium was reported, it cannot be said that the required growth rate is attained at an industrial level even in YPD medium.

Further, the reported γ-glutamylcysteine content of the YL1 strain in which the glutathione synthetase gene was disrupted is as low as 0.533%, and it cannot be accepted for practical use of industrial level (Ootake *et al., Agric. Biol. Chem., supra*). In addition, Chris *et al*. pointed out that since the phenotype of YL1 strain corresponded to that of a strain of which glutathione synthetase was partially reduced, the glutathione synthetase was not fully eliminated from it (Chris M. Grant *et al*., *Molecular Biology of the Cell*, *vol. 8*, pp.1699-1707, 1997). However, since the YL1 strain shows significantly different proliferation abilities during the logarithmic growth phase in a medium containing glutathione and a medium not containing glutathione, it is essentially different from the glutathione synthetase weakened strain of the present invention.

Furthermore, it was reported that glutathione was not detected when glutathione content of the glutathione synthetase gene disrupted strain produced by Inoue *et al.* (*supra*) was measured.

### Summary of the Invention

Under such a technical background as mentioned above, an object of the present invention is to provide a natural food material that can practically be used for improving flavor of foodstuffs like cysteine, more specifically, to provide yeast ,which can be used even for production at industrial level and shows a large accumulation amount of γ-glutamylcysteine, and yeast extract produced by using such yeast.

The inventors of the present invention found that cysteine is released when γ-glutamylcysteine is heated, and conceived that if a natural food material containing γ-glutamylcysteine is heated, a natural food material that can be used like a natural food material containing cysteine could be produced. Therefore, aiming at breeding yeast strains showing a high γ-glutamylcysteine content, the inventors attempted to disrupt the glutathione synthetase gene. However, satisfactory results could not be obtained. The inventors further assiduously studied, and as a result, they successfully obtained a strain showing a high γ-glutamylcysteine content and good growth. Thus, the present invention was accomplished.

That is, the present invention provides the followings.
(1) A strain of *Saccharomyces cerevisiae*, which can contain 1% by weight or more of γ-glutamylcysteine and contains 0.004-0.1% by weight of glutathione during its logarithmic growth phase, when the strain is cultured in a medium in which a glutathione synthetase deficient strain of *Saccharomyces cerevisiae* shows a slower growth rate than a wild type strain.
(2) The strain of *Saccharomyces cerevisiae* according to (1), wherein the medium in which a glutathione synthetase deficient strain of *Saccharomyces cerevisiae* shows a slower growth rate than a wild strain is a medium not containing glutathione or a medium not containing glutathione, γ-glutamylcysteine, L-cysteine and cystine.
(3) The strain of *Saccharomyces cerevisiae* according to (2), wherein the medium is a minimal medium.
(4) A strain of *Saccharomyces cerevisiae,* wherein glutathione synthetase encoded by a glutathione synthetase gene on a chromosome has deletion of a C-terminus region from an arginine residue at a position of 370.
(5) Yeast extract produced by culturing a strain of *Saccharomyces cerevisiae* according to any one of (1) to (4) in a suitable medium and utilizing the obtained cells.
(6) A method for breeding a strain of *Saccharomyces cerevisiae* containing γ-glutamylcysteine, comprising the steps of constructing recombinant strains of *Saccharomyces cerevisiae* in which glutathione synthetase gene is modified by a gene recombination technique and selecting a recombinant strain that contains 0.004-0.1% by weight of glutathione during its logarithmic growth phase when the strain is cultured in a medium in which a glutathione synthetase deficient strain of *Saccharomyces* *cerevisiae* shows a slower growth rate than a wild strain.

The strain of *Saccharomyces cerevisiae* of the present invention produces γ-glutamylcysteine exceeding a certain amount and shows good growth in an industrially used medium such as one not containing glutathione. Therefore, it is useful for efficient production of yeast extract containing γ-glutamylcysteine.

### Brief explanation of the Drawings

Fig. 1 shows liberation of cysteine from γ-glutamylcysteine by heating treatment at pH 3. PCA represents pyrolidonecarboxylic acid, Total Cysteine represents the total amount of cysteine, and γ-Glu-Cys represents γ-glutamylcysteine (the same shall apply to Fig. 2).
Fig. 2 shows liberation of cysteine from γ-glutamylcysteine by heating treatment at pH 5.
Fig. 3 shows construction of plasmid GSH2Mdash/pYES2dash containing a cassette for substitution of weakened-type glutathione synthetase gene (Cassette 2).
Fig. 4 schematically shows gene substitution of glutathione synthetase gene using Cassette 2.
Fig. 5 shows growth of Nα3 strain (OD₆₆₀) in SD medium or SD medium containing 1 mM of glutathione (containing a required amount of uracil).
Fig. 6 shows growth of Nα2 strain and Nα3 strain in SD medium (containing a required amount of uracil).

### Detailed Description of the Invention

Hereafter, the present invention will be explained in detail.

As described above, the present invention is first based on the finding that cysteine is obtained when γ-glutamylcysteine is heated. If γ-glutamylcysteine is heated at 50-120°C for 3 to 300 minutes at pH 1-7, γ-glutamylcysteine is decomposed into cysteine and PCA (pyrolidonecarboxylic acid), and therefore cysteine can be obtained with high yield as a whole. The term "cysteine" may be used hereinafter to refer to both of L-cysteine and cystine, which is an oxidized type disulfide of L-cysteine.

The *Saccharomyces cerevisiae* strain of the present invention is produced based on the aforementioned finding for the purpose of improving flavor of foodstuffs and so forth. When the *Saccharomyces cerevisiae* strain of the present invention is cultured in a medium in which a glutathione synthetase deficient strain of *Saccharomyces cerevisiae* shows a slower growth rate than a wild strain, it contains 1% by weight or more of γ-glutamylcysteine in terms of a ratio with respect to solid components during its logarithmic growth phase. In the present invention, the content of γ-glutamylcysteine or glutathione refers to a content (%) of γ-glutamylcysteine or glutathione with respect to solid components of cells, for example, cell weight after heated at 105°C for 4 hours.

Further, when the *Saccharomyces cerevisiae* strain of the present invention is cultured in a medium in which a glutathione synthetase deficient strain of *Saccharomyces cerevisiae* shows a slower growth rate than a wild strain, it can contain 1% by weight or more, preferably 1.7% by weight or more, of γ-glutamylcysteine and contains 0.004-0.1% by weight, preferably 0.004-0.01% by weight, of glutathione during its logarithmic growth phase. As will be described in examples mentioned hereinafter, the *Saccharomyces cerevisiae* strain of the present invention produces a trace amount of glutathione, and shows growth better than that of a glutathione synthetase deficient strain in a medium that does not contain glutathione. In this specification, a strain which has feeble glutathione synthetase activity in such a degree that it should produce 0.004-0.1% by weight of glutathione in the aforementioned medium, like the *Saccharomyces cerevisiae* strain of the present invention, may also be referred to as "glutathione synthetase weakened strain". On the other hand, a "glutathione synthetase deficient strain" refers to a strain that is substantially deficient in glutathione synthetase activity and cannot produce glutathione in a minimal medium. Further, in the present invention, the term "logarithmic growth phase" refers to a stage during culture in which number of cells of the *Saccharomyces cerevisiae* in culture increases logarithmically to culture time. The γ-glutamylcysteine content need not be always 1% by weight or more during the whole logarithmic growth phase, and it is sufficient that the content becomes 1% by weight or more at any point during the logarithmic growth phase, preferably during such a logarithmic growth phase that culture broth should show an absorbance that corresponds to 1/2 or more of absorbance during stationary phase after the logarithmic growth phase.

The *Saccharomyces cerevisiae* strain of the present invention produces γ-glutamylcysteine in an amount exceeding a certain level and shows good growth in an industrially used medium, for example, a medium not containing glutathione as described above. Therefore, it shows superior productivity of γ-glutamylcysteine per unit time and is suitable for efficient production of yeast extract containing γ-glutamylcysteine. Further, yeast extract of high cysteine content can be produced by heating the obtained yeast extract.

Examples of the medium in which a glutathione synthetase deficient strain of *Saccharomyces cerevisiae* shows a slower growth rate than a wild strain, *i.e*., a strain that has glutathione synthetase activity and produces glutathione, include, for example, a medium not containing glutathione and a medium not containing glutathione, γ-glutamylcysteine, L-cysteine and cystine. Specifically, various kinds of minimal media such as SD medium can be mentioned. When the *Saccharomyces cerevisiae* strain of the present invention shows auxotrophy other than the aforementioned characteristics, the aforementioned medium should contain a nutrient corresponding to such auxotrophy, for example, various amino acids other than cysteine, nucleotides, vitamins and so forth, as required.

Specific examples of the *Saccharomyces cerevisiae* strain of the present invention include a *Saccharomyces cerevisiae* strain that produces a glutathione synthetase having a deletion of a C-terminus region from an arginine residue at a position of 370, *i.e*., a glutathione synthetase of which amino acid residues of 370th position and thereafter are deleted.

Based on the aforementioned report of Inoue *et al.* (Yoshiharu Inoue *et al., Biochimica et Biophysica Acta*, No. 1395, pp.315-320, 1998), it was thought that the glutathione synthetase containing the 1-396th amino acid residues but suffering from deletion of the 397th amino acid residue and the residues thereafter lost the activity. Therefore, it was expected that if any one of the codons of 396th amino acid residue and those upstream therefrom of the glutathione synthetase structural gene was replaced with a stop codon, an expression product would not show the glutathione synthetase activity. However, a gene substituted strain produced by using a glutathione synthetase gene in which the 370th codon was replaced with a stop codon produced a trace amount of glutathione as will be shown in the examples mentioned below, and therefore it was suggested that it had feeble glutathione synthetase activity.

Based on the above finding, the *Saccharomyces cerevisiae* strain of the present invention can be obtained by weakening the glutathione synthetase activity of cells. In order to weaken the glutathione synthetase activity, there can be used a method of changing the promoter of the glutathione synthetase gene from the proper promoter of the gene to a weaker promoter derived from another gene, a method of weakening expression or activity or the both of glutathione synthetase by modifying the promoter or a coding region of glutathione synthetase gene, a method of weakening activity of transcription factor of the gene or the like.

The glutathione synthetase gene sequence can be modified by, for example, usual mutagenesis treatments such as UV irradiation, treatment with a mutagenizing agent such as N-methyl-N-nitrosoguanidine (NTG), ethyl methanesulfonate (EMS), nitrous acid and acridine, or gene substitution utilizing a genetic recombination technique.

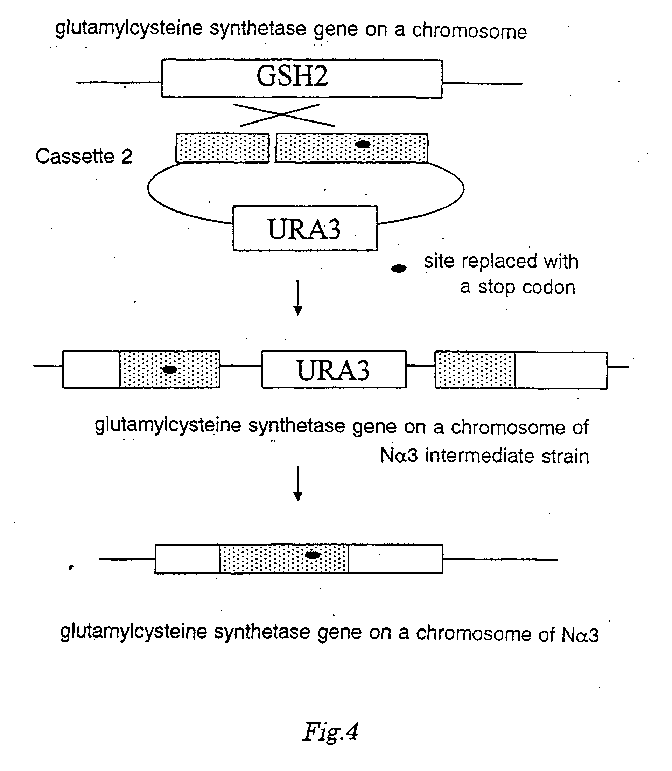

The gene substitution can be performed as follows (see Fig. 4). A *Saccharomyces cerevisiae* strain is transformed with a recombinant DNA containing a glutathione synthetase gene modified so that glutathione synthetase having feeble activity should be encoded (weakened-type glutathione synthetase gene), for example, a glutathione synthetase gene in which the 370th codon is changed to a stop codon, to cause recombination between the weakened-type glutathione synthetase gene and the glutathione synthetase gene on a chromosome. In this case, if a marker gene is included in a plasmid according to a phenotype of host such as auxotrophy, handling will become easy. Further, after the production of the aforementioned recombinant DNA using a plasmid, if it is linearized by digestion with a restriction enzyme and its replication control region which functions in *Saccharomyces cerevisiae* is removed, strains in which the recombinant DNA is taken up into a chromosome can efficiently be obtained.

In a strain in which the recombinant DNA is incorporated into a chromosome as described above, the recombinant DNA causes recombination with a glutathione synthetase gene sequence that originally exists on the chromosome, and two of fused genes of the normal glutathione synthetase gene and the weakened-type glutathione synthetase gene are inserted into the chromosome so that other portions of the recombinant DNA (vector portion and marker gene) should be interposed between them. Therefore, in this state, the normal glutathione synthetase gene functions.

Then, in order to leave only the deletion type glutathione synthetase gene on the chromosome DNA, one copy of the glutathione synthetase gene is dropped from the chromosome DNA together with the vector portion (including the marker gene) by recombination of two of the glutathione synthetase genes. In that case, the normal glutathione synthetase gene is left on the chromosome DNA and the weakened-type glutathione synthetase gene is excised, or conversely, the weakened-type glutathione synthetase gene is left on the chromosome DNA and the normal glutathione synthetase gene is excised. Since a marker gene is excised in any case, the occurrence of the second recombination can be confirmed by examining an expression trait corresponding to the marker gene. Further, the desired gene disrupted strain can be selected by amplifying the glutathione synthetase gene by PCR and investigating its structure.

*Saccharomyces cerevisiae* can be transformed by a method usually used for transformation of yeast, for example, the protoplast method, KU method, KUR method, electroporation method and so forth.

Expression regulatory sequences such as promoters can also be modified in a manner similar to the above. The *Saccharomyces cerevisiae* stain of the present invention may further show enhanced γ-glutamylcysteine synthetase activity, in addition to the feeble glutathione synthetase activity.

The *Saccharomyces cerevisiae* strain of the present invention or a parent strain used for the production thereof may be a haploid, diploid or further higher polyploid.

The *Saccharomyces cerevisiae* strain of the present invention can be obtained by culturing strains of *Saccharomyces cerevisiae* modified as described above in a medium in which a glutathione synthetase deficient strain of *Saccharomyces cerevisiae* shows a slower growth rate than a wild strain and selecting a recombinant strain containing glutathione in the range of 0.004-0.1% by weight during its logarithmic growth phase.

Yeast extract containing γ-glutamylcysteine can be produced by culturing the *Saccharomyces cerevisiae* strain of the present invention in a suitable medium and using the obtained cells. Further, by heating the obtained yeast extract, yeast extract with a high cysteine content can be produced.

The medium used for the production of yeast extract is not particularly limited, so long as the *Saccharomyces cerevisiae* strain of the present invention shows good growth and efficiently produces γ-glutamylcysteine in it. In particular, since the *Saccharomyces cerevisiae* strain of the present invention can show good growth even in a medium not containing glutathione, a medium usually used for industrial purpose can be used. Necessary nutrients are further added to the medium as required depending on traits of a strain to be used.

Culture conditions and procedure for the preparation of yeast extract may be similar to those for usual culture of *Saccharomyces cerevisiae* and preparation of yeast extract. The yeast extract may be prepared by treating an extract obtained from extraction of yeast cells with hot water or treating digested yeast cells.

### Best Mode for Carrying out the Invention

Hereafter, the present invention will be explained more concretely with reference to the following examples.

### <1> Liberation of cysteine from γ-glutamylcysteine by heat treatment

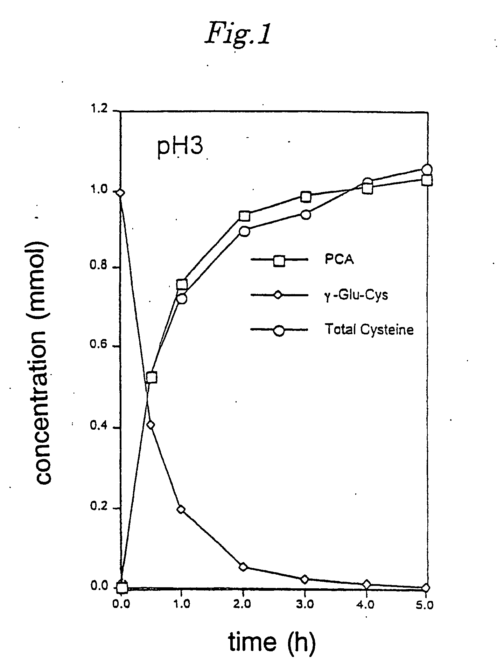

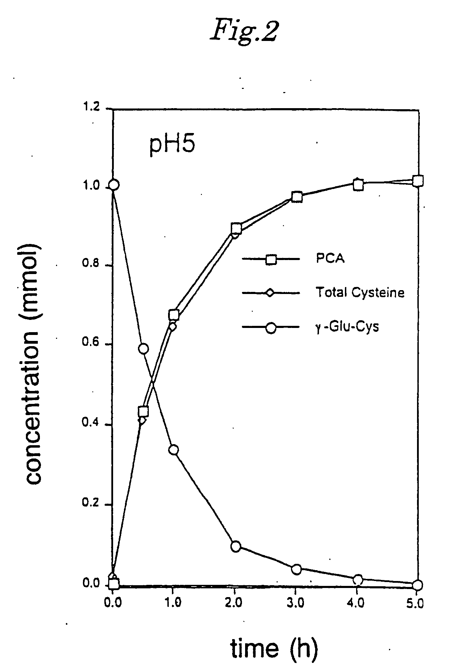

An aqueous solution of reduced-type γ-glutamylcysteine at a concentration of 1 mmol (pH was adjusted to 3 or 5) was heated at 98°C, and products were investigated in the time course. As a result, it was found that, as shown in Figs. 1 and 2, γ-glutamylcysteine was decomposed into cysteine and pyrolidonecarboxylic acid (shown as "PCA" in Figs. 1 and 2) by heating, and cysteine could be obtained with high yield.

### <2> Construction of glutathione synthetase gene disrupted strain

Then, a glutathione synthetase gene disrupted strain was constructed.

### (1) Isolation of Saccharomyces cerevisiae showing uracil auxotrophy

In a conventional manner, a haploid Nα strain was obtained from *Saccharomyces cerevisiae* isolated from the nature. An Nα1 strain showing uracil auxotrophy was obtained from the Nα strain using an SDFOA plate containing uracil (SD medium containing 2% of purified agar, 50 mg/L of uracil and 1 g/L of 5-fluoroorotic acid hydrate as the final concentrations). Since the uracil auxotrophy of the Nα1 strain was complemented with the URA3 gene as will be described later, the strain was considered to be a variant strain for the URA3 gene.

| (Composition of SD medium) | |
|---|---|
| Glucose | 2% |
| Nitrogen Base | 1-fold concentration |

(Nitrogen Base of 10-fold concentration was prepared by dissolving a mixture of 1.7 g Bacto Yeast Nitrogen Base w/o Amino Acids and Ammonium Sulfate (Difco) and 5 g of ammonium sulfate in 100 ml of sterilized water, adjusting the solution to about pH 5.2, and subjecting the solution to filtration sterilization using a filter)

### (2) Production of cassette for glutathione synthetase deficiency

A glutathione synthetase gene disrupted strain was constructed by using the Nα1 strain as a parent strain.

First, a region from the upstream region to the terminus region of the glutathione synthetase (GSH2) gene was amplified by PCR using chromosome DNA of the Nα1 strain as a template. PCR was performed by allowing a reaction at 94°C for 1 minute and then repeating 30 times a cycle consisting of reactions at 94°C for 30 seconds, 60°C for 40 seconds and 74°C for 1 minute and 30 seconds using a reaction solution having the following composition.

| (Composition of reaction solution for PCR) | |
|---|---|
| Solution of chromosome DNA | 1 µl |
| 10X PCR buffer | 10 µl |
| 10 mM dNTPs | 10 µl |
| 10 pmol/µl GAL11F (SEQ ID NO: 1) | 1 µl |
| 10 pmol/µl GSH2R3 (SEQ ID NO: 2) | 1 µl |
| Purified water | 76 µl |
| KOD Dash (TOYOBO) * | 1 µl |
| Total | 100 µl |

| | |
|---|---|
| (*: polymerase for PCR) | |

The GSH2 gene fragment amplified as described above was ligated to a plasmid pGEM-T Easy (Promega) according to the manufacturer's instruction to obtain GSH2/pGEM.

Separately, the URA3 gene was obtained as a selection gene marker by PCR using a plasmid pYES2 (Invitrogen) containing the gene as a template. PCR was performed by allowing a reaction at 94°C for 1 minute and then repeating 30 times a cycle consisting of reactions at 94°C for 30 seconds, 52°C for 30 seconds and 74°C for 40 seconds using a reaction solution having the following composition.

| (Composition of reaction solution for PCR) | |
|---|---|
| 10 ng/µl pYES2 | 1 µl |
| 10X PCR buffer | 10 µl |
| 10 mM dNTPs | 10 µl |
| 10 pmol/µl URA3F2 (SEQ ID NO: 3) | 1 µl |
| 10 pmol/µl URA3R2 (SEQ ID NO: 4) | 1 µl |
| Purified water | 76 µl |
| KOD Dash | 1 µl |
| Total | 100 µl |

Then, GSH2/pGEM was digested with a restriction enzyme *Mun*I, and the termini were blunt-ended. To the digested ends, an URA3 gene fragment of which ends were blunt-ended with a restriction enzyme *Sma*I was ligated to prepare a plasmid URA3-GSH2/pGEM. PCR was performed by using this URA3-GSH2/pGEM as a template and primers having sequences corresponding to the end regions of the GSH2 gene to prepare Cassette 1. PCR was performed by allowing a reaction at 94°C for 1 minute and then repeating 30 times a cycle consisting of reactions at 94°C for 30 seconds, 56°C for 30 seconds and 74°C for 1 minute using a reaction solution having the following composition.

| (Composition of reaction solution for PCR) | |
|---|---|
| 10 ng/µl URA3-GSH2/pGEM | 1 µl |
| 10X PCR buffer | 10 µl |
| 10 mM dNTPs | 10 µl |
| 10 pmol/µl GAL11F (SEQ ID NO: 1) | 1 µl |
| 10 pmol/µl GSH2R (SEQ ID NO: 5) | 1 µl |
| Purified water | 76 µl |
| KOD Dash | 1 µl |
| Total | 100 µl |

### (3) Acquisition of glutathione synthetase gene deficient strain

The glutathione synthetase gene of the Nα1 strain was disrupted by using Cassette 1 produced as described above. The Nα1 strain was precultured, and the culture was subcultured in 50 ml of YPD medium until the culture reached the logarithmic growth phase. The cultured cells were suspended in 1 M sorbitol and mixed with Cassette 1, and transformation was performed by electroporation. Transformant strains were cultured on SD plates containing 1 mM of glutathione, and grown strains were selected. By PCR and measurement of glutathione content in cells as described later, a strain of which glutathione synthetase gene was replaced with Cassette 1 was selected to obtain Nα2 strain.

In the Nα2 strain produced as described above, a sequence derived from the URA3 gene fragment was added after the 11th codon in the cording region of the glutathione synthetase gene. Therefore, the glutathione synthetase gene was correctly translated only for a sequence up to the 11th amino acid residue.

### <3> Construction of glutathione synthetase weakened strain

Then, a strain having substitution of weakened-type glutathione synthetase gene was produced.

### (1) Production of cassette for substitution of weakened-type glutathione synthetase gene

The glutathione synthetase gene fragment of the Nα1 strain was amplified by PCR. PCR was performed by allowing a reaction at 98°C for 10 seconds and then repeating 30 times a cycle consisting of reactions at 98°C for 10 seconds, 60°C for 30 seconds and 72°C for 1 minute using a reaction solution having the following composition.

| (Composition of reaction solution for PCR) | |
|---|---|
| Yeast chromosome | 1 µl |
| Pyrobest DNA Polymerase (Takara Shuzo) | 0.5 µl |
| 10X PCR buffer | 10 µl |
| 10 mM dNTPs | 8 µl |
| 20 pmol/µl GSH2F7 (SEQ ID NO: 6) | 2 µl |
| 20 pmol/µl GSH2R7 (SEQ ID NO: 7) | 2 µl |
| Purified water | 76.5 µl |
| Total | 100 µl |

The gene fragment amplified as described above was purified, and nucleotides A were added to its end by an enzymatic reaction performed at 72°C for 10 minutes in a reaction solution having the following composition.

| (Composition of reaction solution) | |
|---|---|
| Solution of gene fragment | 5 µl |
| 10X PCR buffer (MgCl ₂ free) | 10 µl |
| 25 mM MgCl ₂ | 3 µl |
| 2.5 mM dATP | 5 µl |
| Taq DNA polymerase (Takara Shuzo) | 0.5 µl |
| Purified water | 31.5 µl |
| Total | 50 µl |

The reaction product was ligated to a plasmid pGEM-T Easy (Promega) according to the manufacturer's instruction to obtain a plasmid GSH2dash/pGEM.

Then, the codon corresponding to the 370th amino acid of the glutathione synthetase gene contained in GSH2dash/pGEM was replaced with a stop codon by site-specific mutgenesis. This procedure was performed by using QuikChange™ Site-Directed Mutagenesis Kit (STRATAGENE) according to the manufacturer's instruction. As the primers, GSH2M-F1 (SEQ ID NO: 8) and GSH2M-R1 (SEQ ID NO: 9) were used. Thus, a plasmid GSH2Mdash/pGEM was produced.

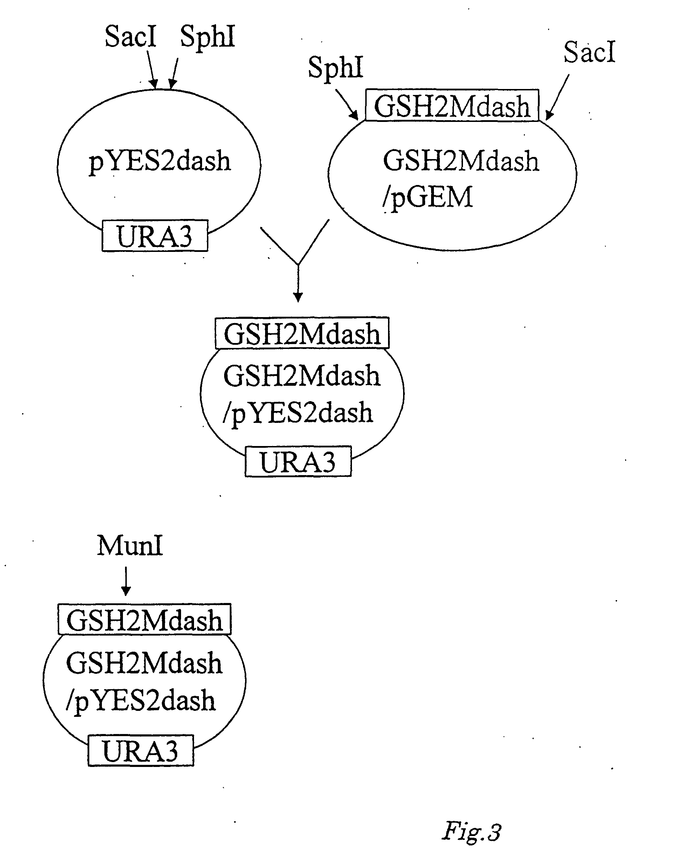

Separately, a plasmid corresponding to the plasmid pYES2 (Invitrogen) of which 2µori was removed was produced. pYES2 was digested with restriction enzymes SspI and NheI, the digested ends were blunt-ended, and the products were ligated to obtain a plasmid pYES2dash. Each of pYES2dash and GSH2Mdash/pGEM was digested with restriction enzymes SacI and SphI to obtain a fragment containing URA3 gene from pYES2dash and a glutathione synthetase gene fragment having a mutation from GSH2Mdash/pGEM, and these fragments were ligated. Thus, a plasmid GSH2Mdash/pYES2dash was produced. GSH2Mdash/pYES2dash was digested with a restriction enzyme MunI to obtain Cassette 2 (Fig. 3).

### (2) Construction of strain having weakened-type glutathione synthetase gene substitution

Gene substitution of the glutathione synthetase gene of the Nα1 strain was performed by using Cassette 2 produced as described above (Fig. 4). The Nα1 strain was precultured, and the culture was subcultured in 50 ml of YPD medium until the culture reached the logarithmic growth phase. The cultured cells were suspended in 1 M sorbitol and mixed with Cassette 2, and transformation of the cells was attained by electroporation. The transformant strains are cultured on an SD plate containing 1 mM of glutathione, and the grown strains were selected. Incorporation of Cassette 2 at the desired site on the chromosome was confirmed by PCR, and the obtained strain was designated as Nα3 intermediate strain.

Then, the following procedures were performed in order to leave only the weakened-type glutathione synthetase gene on the chromosome as shown in Fig. 4. The Nα3 intermediate strain was cultured in YPD medium, and the culture product was inoculated on an SDFOA plate containing 1 mM of glutathione. The sequence of the glutathione synthetase gene of a strain grown on the plate was determined to confirm the sequence of the target site was correctly substituted. Thus, an Nα3 strain was obtained.

### <4> Growth of Nα2 strain and Nα3 strain and production of γ-glutamylcysteine

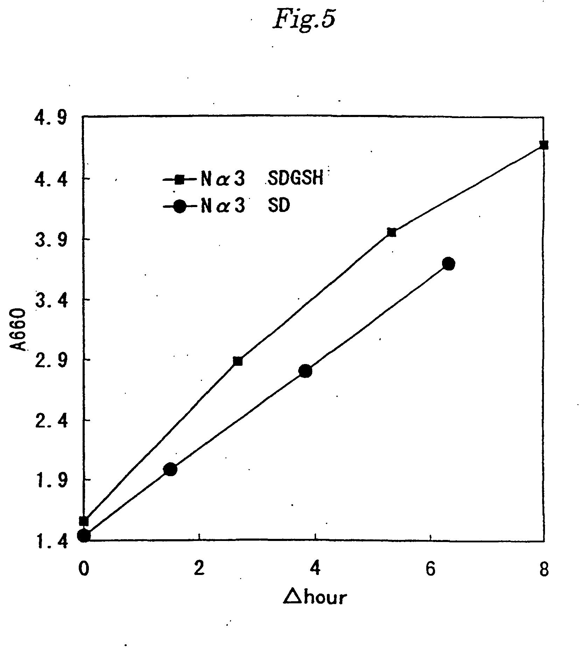

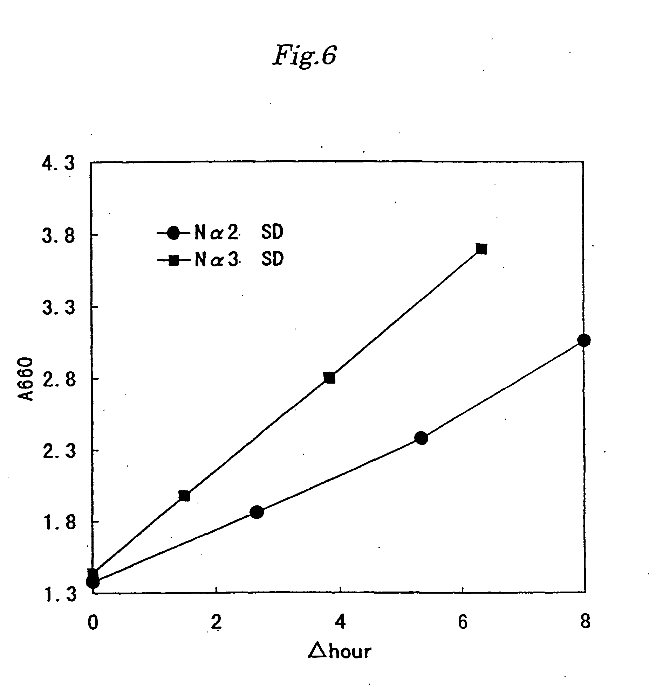

Proliferation ability in the logarithmic growth phase of the Nα2 strain and the Nα3 strain obtained as described above was investigated. The Nα2 strain and the Nα3 strain were precultured in YPD medium, and the cultures were each inoculated in 50 ml of SD medium (containing 50 mg/L of uracil) or SD medium (containing 50 mg/L of uracil) containing 1 mM of glutathione, and cultured at 30°C with shaking. The results are shown in Figs. 5 and 6. As shown in Fig. 5, the Nα3 strain did not show significant difference of proliferation ability in the medium not containing glutathione compared with the medium containing glutathione. Further, the Nα3 strain showed better growth in the logarithmic growth phase in the medium not containing glutathione compared with the Nα2 strain (Fig. 6).

Then, production amounts of γ-glutamylcysteine and glutathione per unit time in the logarithmic growth phase were investigated for the Nα2 strain and the Nα3 strain. The Nα2 strain and the Nα3 strain were precultured in YPD medium, and the cultures were each inoculated in 50 ml of SD medium (containing a required amount of uracil), and cultured at 30°C with shaking.

The production amounts of γ-glutamylcysteine and glutathione were measured as follows. Cells were collected by centrifugation of each culture, and the cells were washed twice with distilled water and extracted with hot wafer at 70°C for 10 minutes to obtain cell content. The cell content was centrifuged, and γ-glutamylcysteine and glutathione contents in the obtained supernatant were measured. Further, yeast cells contained in a predetermined amount of medium was taken on filter paper, and heated at 105°C for 4 hours. Then, the remained cells were weighed and the weight was used as dry cell weight. Contents of γ-glutamylcysteine and glutathione per dry cell weight are shown in Table 1.

**Table 1**

| | Culture time before measurement** | γ-Glutamylcysteine (%) | Glutathione (%) |
|---|---|---|---|
| Nα2 strain No.1 | About 2.6 hours | 1.752 | 0 |
| Nα2 strain No.2 | About 5.3 hours | 1.748 | 0 |
| Nα3 strain No.1 | About 1.5 hours | 1.101 | 0.0043 |
| Nα3 strain No.2 | About 3.8 hours | 1.117 | 0.0045 |

From these results, γ-glutamylcysteine production amount per unit time was calculated for each strain. In order to demonstrate the preference of the Nα3 strain to the Nα2 strain, results for the strain showing a higher γ-glutamylcysteine content (Nα2 strain No.1) among the Nα2 strains and the strain showing a lower γ-glutamylcysteine content (Nα3 strain No.1) among the Nα3 strains were used for the calculation. That is, a maximum value was calculated for the Nα2 strain, and a minimum value for the Nα3 strain. As a result, the production amounts were 0.116 mg/hour for the Nα2 strain and 0.124 mg/hour for the Nα3 strain.

## Claims

1. A strain of *Saccharomyces cerevisiae*, which can contain 1% by weight or more of γ-glutamylcysteine and contains 0.004-0.1% by weight of glutathione during its logarithmic growth phase, when the strain is cultured in a medium in which a glutathione synthetase deficient strain of *Saccharomyces cerevisiae* shows a slower growth rate than a wild strain.

2. The strain of *Saccharomyces cerevisiae* according to claim 1, wherein the medium in which a glutathione synthetase deficient strain of *Saccharomyces cerevisiae* shows a slower growth rate than a wild strain is a medium not containing glutathione or a medium not containing glutathione, γ-glutamylcysteine, L-cysteine and cystine.

3. The strain of *Saccharomyces cerevisiae* according to claim 2, wherein the medium is a minimal medium.

4. A strain of *Saccharomyces cerevisiae*, wherein glutathione synthetase encoded by a glutathione synthetase gene on a chromosome has deletion of a C-terminal region from an arginine residue at a position of 370.

5. Yeast extract produced by culturing a strain of *Saccharomyces cerevisiae* according to any one of claims 1-4 in a suitable medium and utilizing the obtained cells.

6. A method for providing a Saccharomyces cerevisiae strain having a weakened glutathione synthetase activity of cells, wherein said Saccharomyces cerevisiae strain contains glutathione within the range of 0.004-0.1% by weight of glutathione during its logarithmic growth phase when the strain is cultured in a medium in which a glutathione synthetase deficient strain of *Saccharomyces cerevisiae* shows a slower growth rate than a wild strain, comprising the step of :
weakening the activity of glutathione sythetase of the strain by modifying a promoter sequence or a coding region of the glutathione synthetase gene.

## Patentansprüche

1. *Saccharomyces cerevisiae*-Stamm, welcher 1 Gew.-% oder mehr γ-Glutamylcystein enthalten kann und 0,004-0,1 Gew.-% Glutathion während dessen logarithmischer Wachstumsphase enthält, wenn der Stamm in einem Medium kultiviert wird, in welchem ein Glutathionsynthetase-defizienter Stamm von *Saccharomyces cerevisiae* eine langsamere Wachstumsgeschwindigkeit als ein Wildstamm zeigt.

2. *Saccharomyces cerevisiae*-Stamm nach Anspruch 1, wobei das Medium, in welchem ein Glutathionsynthetase-defizienter Stamm von *Saccharomyces cerevisiae* eine langsamere Wachstumsgeschwindigkeit als ein Wildstamm zeigt, ein Medium ist, welches Glutathion nicht enthält, oder ein Medium, welches Glutathion, γ-Glutamylcystein, L-Cystein und Cystin nicht enthält.

3. *Saccharomyces cerevisiae*-Stamm nach Anspruch 2, wobei das Medium ein Minimalmedium ist.

4. *Saccharomyces cerevisiae*-Stamm, in dem die Glutathionsynthetase, die durch ein Glutathionsynthetasegen auf einem Chromosom kodiert wird, eine Deletion eines C-terminalen Bereichs ab einem Arigininrest an Position 370 aufweist.

5. Hefeextrakt, der durch Kultivieren eines *Saccharomyces cerevisiae*-Stamms nach einem der Ansprüche 1 bis 4 in einem geeigneten Medium unter Verwendung der erhaltenen Zellen kultiviert wird.

6. Verfahren zur Bereitstellung eines *Saccharomyces cerevisiae*-Stamms mit abgeschwächter Glutathionsynthetase-Aktivität von Zellen, wobei der *Saccharomyces cerevisiae*-Stamm Glutathion in dem Bereich von 0,004-0,1 Gew.-% Glutathion während dessen logarithmischer Wachstumsphase enthält, wenn der Stamm in einem Medium kultiviert wird, in welchem ein Glutathionsynthetase-defizienter Stamm von *Saccharomyces cerevisiae* eine langsamere Wachstumsgeschwindigkeit als ein Wildstamm zeigt, umfassend den Schritt:
Abschwächen der Glutathionsynthetase-Aktivität des Stamms durch Modifizieren einer Promotersequenz oder eines kodierenden Bereichs des Glutathionsynthetasegens.

## Revendications

1. Souche de *Saccharomyces cerevisiae*, qui peut contenir 1 % en poids ou plus de γ-glutamylcystéine et qui contient 0,004 à 0,1 % en poids de glutathion au cours de sa phase de croissance logarithmique, lorsque la souche est cultivée dans un milieu dans lequel une souche de *Saccharomyces cerevisiae* déficiente en glutathion synthétase présente un taux de croissance plus lent qu'une souche sauvage.

2. Souche de *Saccharomyces cerevisiae* selon la revendication 1, dans laquelle le milieu dans lequel une souche de *Saccharomyces cerevisiae* déficiente en glutathion synthétase présente un taux de croissance plus lent qu'une souche sauvage est un milieu ne contenant pas de glutathion ou un milieu ne contenant pas de glutathion, de γ-glutamylcystéine, de L-cystéine et de cystine.

3. Souche de *Saccharomyces cerevisiae* selon la revendication 2, dans laquelle le milieu est un milieu minimal.

4. Souche de *Saccharomyces cerevisiae*, dans laquelle la glutathion synthétase codée par un gène de la glutathion synthétase sur un chromosome a une délétion d'une région C-terminale à partir d'un résidu arginine en position 370.

5. Extrait de levure produit en cultivant une souche de *Saccharomyces cerevisiae* selon l'une quelconque des revendications 1 à 4 dans un milieu adapté et en utilisant les cellules obtenues.

6. Procédé pour fournir une souche de *Saccharomyces cerevisiae* ayant une activité glutathion synthétase des cellules affaiblie, dans laquelle ladite souche de *Saccharomyces cerevisiae* contient du glutathion dans le domaine allant de 0,004 à 0,1 % en poids de glutathion au cours de sa phase de croissance logarithmique lorsque la souche est cultivée dans un milieu dans lequel une souche de *Saccharomyces cerevisiae* déficiente en glutathion synthétase présente un taux de croissance plus lent qu'une souche sauvage, comprenant les étapes consistant à :
- affaiblir l'activité glutathion synthétase de la souche en modifiant une séquence promoteur ou une région codante du gène de la glutathion synthétase.
